# EUROPEAN PATENT SPECIFICATION

(11) **EP 3 768 338 B1**
(45) Date of publication and mention of the grant of the patent: **05.08.2026**
(21) Application number: 18910255.1
(22) Date of filing: 12.05.2018
(51) Int. Cl.: A61L 29/14, A61L 29/08, A61L 29/16

(54) **DRUG COATED BALLOON**
MEDIKAMENTENBESCHICHTETER BALLON
BALLONNET REVÊTU DE MÉDICAMENT

(30) Priority: 21.03.2018 IN 201821010337
(43) Date of publication of application: 27.01.2021
(73) Proprietor: Meril Life Sciences Pvt Ltd, Gujarat, Vapi 396191 (IN)
(72) Inventor: KOTHWALA, Deveshkumar Mahendralal, Surat, Gujarat 395003 (IN); VYAS, Rajnikant Gandalal, Powai, Mumbai 400076 (IN); MINOCHA, Pramod Kumar, Vapi, Gujarat 396191 (IN)
(74) Representative: Marks & Clerk LLP
(86) International application number: PCT/IN2018/050300
(87) International publication number: WO 2019/180725

(56) References cited:
- WO-A1-2014/143048
- US-A1- 2010 324 645
- US-A1- 2011 144 578
- US-A1- 2011 144 582
- US-A1- 2016 058 915

## Description

### FIELD OF INVENTION

The present invention relates to a medical device, more specifically the present invention relates to a drug coated balloon.

### BACKGROUND

Peripheral artery disease (PAD) is a disease in which plaque builds up in the arteries that carry blood to head, organs and limbs. PAD usually affects the arteries of the legs however it can also affect the arteries that carry blood from heart to head, kidneys, arms, and stomach. The treatment of peripheral artery disease is usually done with the help drug eluting stents. A drug eluting stent is a peripheral or coronary stent which is placed in a narrow, diseased artery such that the stent slowly releases a drug to block cell proliferation. The drug eluting stents are either polymer stents or rigid metal stents. However, the drug eluting stents have some limitations which include late stent thrombosis that carries with it a high mortality rate. In case of uneven stent expansion or metal stent fracture, vessel injury and/or stent restenosis may occur.

Instead of using the drug eluting stents, a drug coated balloon catheter can be used for the treatment of PAD. A drug coated balloon is a conventional semicompliant angioplasty balloon covered with an anti-proliferative drug which is released into the vessel wall during inflation of the balloon. Small amount of drug is left behind in the vessel wall that is less likely to induce unfavorable response to foreign materials and allows for complete restoration of vessel wall. The use of drug coated balloons (DCB) has an advantage when compared to drug eluting stents (DES) of shortened duration of anti-platelet therapy. Further, the benefits of drug coated balloon include short exposure of the drug coated balloon to the vessel wall.

Most of the drug coated balloons presently available may have an uneven coating surface. The coating layer may be very dry which results in surface damage and in turn drug loss during the removal of balloon catheter prior to implantation.

A coating composition for a balloon is disclosed in a prior art having publication number US20110144578A1. The said composition includes a hydrophobic therapeutic agent and an emulsifier that is solid at ambient temperature. The said composition is heat treated over a balloon resulting in dryness. This further lead to formation of flakes during balloon processing and loss of therapeutic drug during navigation of the balloon to the treatment site.

Another therapeutic formulation for balloon coating is disclosed in a prior art having publication number WO2014143048A1. The said formulation includes a therapeutic agent and an adhesion additive. However, the said formulation will not be suitable for pleat and folding of balloon as it will compromise integrity of the coating. This will further lead to non-uniform dispersion of drug on the balloon surface resulting in reduced therapeutic effect.

The present invention deals with the coating and manufacturing of the drug coated balloon (DCB) to overcome the above mentioned drawbacks.

### SUMMARY

In accordance with an aspect of the invention there is provided a drug coated balloon according to claim 1.

The present invention discloses a drug coated balloon. The drug coated balloon includes a balloon having an outer surface coated with a single layer of a solution of an anti-proliferative drug. The single layer includes at least one antiproliferative drug in the concentration of 60-70 weight % and at least two excipients. The at least two excipients include urea present in the concentration of 30-35 weight % and at least one plasticizer present in the concentration of 3-6 weight % .

Urea disperses the anti-proliferative drug from a surface of the balloon and prevents flaking of the single layer of the anti-proliferative drug upon dispersion, wherein the at least one plasticizer prevents excessive loss of at least one anti-proliferative drug during transit of the balloon from a puncture site to a treatment site, the at least one plasticizer prevents the single layer of the antiproliferative drug from drying.

In alternate non-claimed embodiment, the drug coated balloon includes a base layer, a top layer and a middle layer sandwiched between the base layer and the top layer. The base and top layers include at least one plasticizer in the concentration of 8-12 weight % and at least one adhesive binder in the concentration of 0.5-0.8 weight %. The middle layer includes a solid-lipid nanoparticle suspension of at least one anti-proliferative drug, at least one emulsifier and at least one stabilizer in the concentrations of 40-60 weight %, 15-20 weight % and 1-7 weight % respectively.

The at least one plasticizer prevents excessive loss of at least one antiproliferative drug during transit of the balloon from a puncture site to a treatment site and prevents a single layer of the anti-proliferative drug from getting dried. The at least one adhesive binder ensures adequate retention of the coating formulation over the balloon surface. The at least one emulsifier helps in achieving higher drug encapsulation efficiency and higher cellular uptake. The at least one stabilizer helps in increasing the drug encapsulation efficiency.

### BRIEF DESCRIPTION OF THE DRAWINGS

The summary above, as well as the following detailed description of illustrative embodiments, is better understood when read in conjunction with the appended drawings. For the purpose of illustrating the present disclosure, exemplary constructions of the disclosure are shown in the drawings. However, the disclosure is not limited to specific methods and instrumentalities disclosed herein. Moreover, those in the art will understand that the drawings are not to scale.
Fig. 1 represents a balloon catheter in accordance with an embodiment of the present invention.
Fig. 2 represents a single layer coating on the surface of a balloon of the balloon catheter of FIG. 1 in accordance with an embodiment of the present invention.
Fig. 3 represents a multi-layer coating on the surface of a balloon of the balloon catheter of FIG. 1 in accordance with a non-claimed embodiment of the present disclosure.
Fig. 4 depicts an apparatus used to prepare Solid-Lipid Nano-particle suspension.
Fig. 5 (a) illustrates a flow chart depicting the process of coating of a single layer on the outer surface of the balloon in accordance with an embodiment of the present invention.
Fig. 5 (b) illustrates a flow chart depicting the process of coating of three layers on the outer surface of the balloon in accordance with a non-claimed embodiment of the present disclosure.

### DETAILED DESCRIPTION OF THE DRAWINGS

Prior to describing the invention in detail, definitions of certain words or phrases used throughout this patent document will be defined: the terms "include" and "comprise", as well as derivatives thereof, mean inclusion without limitation; the term "or" is inclusive, meaning and/or; the phrases "coupled with" and "associated therewith", as well as derivatives thereof, may mean to include, be included within, interconnect with, contain, be contained within, connect to or with, couple to or with, be communicable with, cooperate with, interleave, juxtapose, be proximate to, be bound to or with, have a property of, or the like; Definitions of certain words and phrases are provided throughout this patent document, and those of ordinary skill in the art will understand that such definitions apply in many, if not most, instances to prior as well as future uses of such defined words and phrases.

Particular embodiments of the present disclosure are described herein below with reference to the accompanying drawings, however, it is to be understood that the disclosed embodiments are merely examples of the disclosure, which may be embodied in various forms. Well-known functions or constructions are not described in detail to avoid obscuring the present disclosure in unnecessary detail. Therefore, specific structural and functional details disclosed herein are not to be interpreted as limiting, but merely as a basis for the claims and as a representative basis for teaching one skilled in the art to variously employ the present disclosure in virtually any appropriately detailed structure.

In accordance with the present disclosure, a drug coated balloon, coated with one or more layers, is disclosed. The drug coated balloon is utilized for the treatment of diseases for example, a peripheral artery disease. The drug coated balloon is deployed at a treatment site with the help of a balloon catheter. Subsequently, the drug coated balloon is expanded at the treatment site thereby releasing a drug once in contact with an arterial wall. In an embodiment, the components of the layers include one or more anti-proliferative drugs, one or more binders, one or more plasticizers, one or more emulsifiers, one or more salts, one or more humectants, one or more stabilizers dissolved in one or more solvents. The present invention provides uniform coating on the surface of the balloon thus ensuring minimal loss of drug during drug delivery at the treatment site.

The uniform coating as disclosed in the present invention results in maximum amount of drug uptake at the treatment site for tissue. The coating is applied in a way such that no coating damage occurs during assembly of the balloon or after the sterilization of the balloon. Assembly of the balloon includes without limitation folding of the balloon, pleating of the balloon, mounting of the balloon on a balloon catheter, etc. The components of the coating in the present invention are easily dispersed in the solvent(s) without any flaking. The drug coated balloon of the present invention confers minimal or no damage to the applied coating during the pleat-fold process. The parameter of pleat-fold process ensures a low profile drug coated balloon while the coating integrity remains intact.

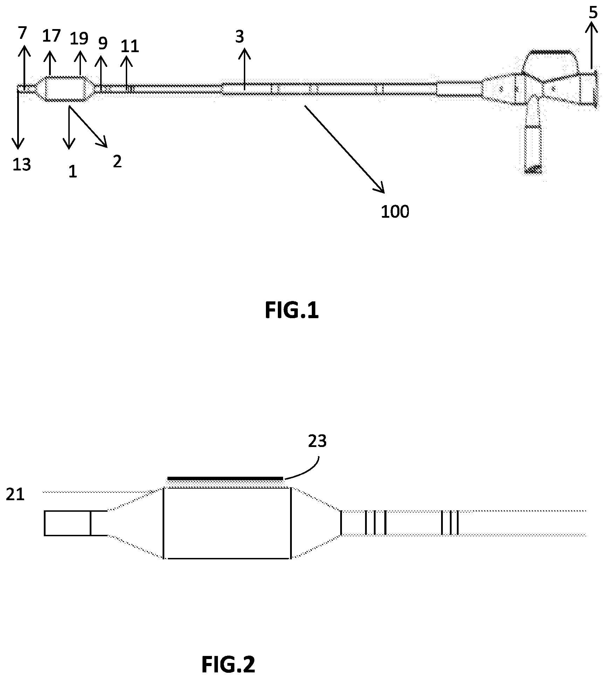

Now referring specifically to the drawings, FIG.1 represents an exemplary schematic view of a balloon catheter 100. As represented, the balloon catheter 100 includes without limitation a balloon 1, a shaft 3, an inner tube (not shown), a proximal end 5, a distal end 7, one or more radiopaque marker bands 9 and 11 and a soft-tip 13. In an embodiment, the balloon catheter 100 utilizes an over the wire (OTW) 0.035" percutaneous transluminal angioplasty (PTA) balloon.

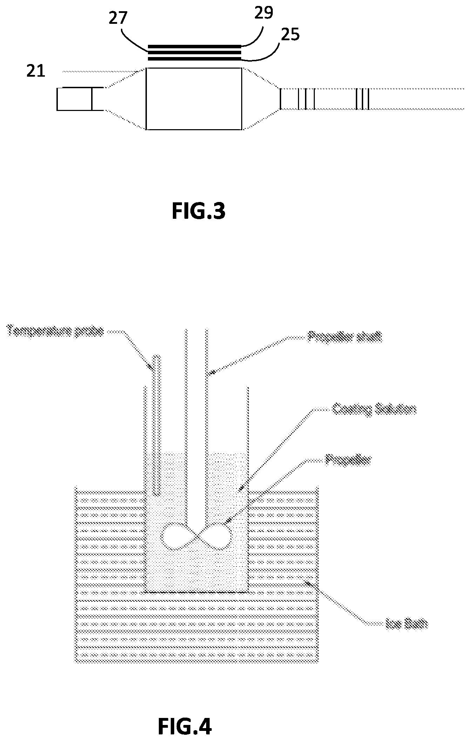

The balloon 1 includes a middle portion 15, a distal end 17 and a proximal end 19. In an embodiment, the distal end 17 and the proximal end 19 of the balloon 1 are tapered. Further, the balloon 1 has an outer surface 21. The outer surface 21 of the balloon 1 may be coated with one or more layers of drug (explained as layers 23, 25, 27 and 29 in FIG.2 and FIG.3). The uniform coating on the outer surface of the balloon ensures minimal loss of drug during drug delivery at the treatment site.

In an embodiment, the balloon 1 may be made of a polymeric material. The polymeric material may include without limitation nylon, pebax, polyethylene terephthalate, polyurethane etc. In an embodiment, the balloon 1 is made of semi compliant pebax. The balloon 1 may be, without limitation, a percutaneous transluminal angioplasty balloon (PTA), a percutaneous transluminal valvuloplasty (PTV), a percutaneous transluminal coronary angioplasty (PTCA balloon), a tapered balloon, a pediatric balloon, etc.

The balloon 1 is mounted on the shaft 3 of the balloon catheter 100. The balloon 1 may expand when an inflation fluid or inflation pressure is applied to the balloon 1 via a hub provided at the proximal end 5 of the balloon catheter 100. During balloon expansion at the treatment site, the distal end 17 and the proximal end 19 of the balloon 1 do not come in contact with an arterial tissue wall while the drug is transferred to the underlying tissue.

The shaft 3 is a hollow tubular structure. The shaft 3 may be made of, without limitation polymeric materials or metals. The shaft 3 has a hollow portion, a distal end and a proximal end. The distal end of the shaft 3 is coupled to the proximal end of the balloon 1. The hollow portion of shaft 3 extends from the proximal end to the distal end. The hollow portion provides a passage way for components like inner tube, inflation fluid, etc. The shaft 3 also permits the movement of a guide wire.

The shaft 3 is provided with a plurality of holes in the region where the balloon 1 is mounted. These holes are enclosed by a middle portion of the balloon 1. The holes on the shaft 3 may either be circular or non-circular in shape. The holes may be arranged in the form of an array on the shaft 3 or may be arranged randomly. The holes are provided to inflate or deflate the balloon 1 by injecting or retrieving a fluid (for example, saline) provided via the hollow portion.

The radiopaque marker bands 9 and 11 can be optionally provided in the balloon catheter 100. As an example, the radiopaque marker bands 9 and 11 are placed near the distal end 7 of the balloon catheter 100. Though in the context of the invention two radiopaque marker bands are described, it is possible that more or lesser number of makers may be used. The radiopaque marker bands 9 and 11 help to position the balloon 1 during deployment of the balloon 1 inside a diseased artery.

The balloon catheter 100 preferably has a soft-tip 13. The soft tip 13 is mounted on the shaft 3 and is disposed at the distal end 7 of the balloon catheter 100. The soft tip 13 is preferably formed from a material such as, for example, a plastic resin, polyethylene, polyvinylchloride, silicone, or Teflon. The shaft 3, the inner tube and the soft tip 13 may be heat welded at the distal end 7 of the balloon catheter 100. The soft tip 13 is configured to guide the shaft 3 through a patient's body lumen while avoiding perforation of the patient's body lumen.

FIG.2 depicts the balloon 1 coated with a layer 23 of a formulation of an antiproliferative drug. The layer 23 as depicted is a single layer. The single layer of coating ensures fast and uniform dispersion of the drug from the outer surface 21 of the balloon 1.

The layer 23 may be formulated in a defined proportion. The formulation of the layer 23 may include one or more of a solvent, one or more anti-proliferative drugs and one or more excipients. The anti-proliferative drug(s) may be in the range of 60-70 weight %, while the excipient(s) may be in the range of 30-35 weight %.

The solvent(s) provides a base for mixing anti-proliferative drug(s) and excipient(s). The solvent(s) may be selected from one or more of, without limitation water, acetone, dichloro methane, alcohols (like methyl alcohol, ethyl alcohol, propyl alcohol, isopropyl alcohol, dimethyl formamide, dimethyl sulfoxide or mixtures thereof.

The anti-proliferative drug(s) may be selected from one or more of, without limitation, sirolimus, biolimus, zotarolimus, everolimus, paclitaxel, vinblastine, vindesine or vincristine. In an embodiment, paclitaxel may be used as the antiproliferative drug. The dose of paclitaxel used may range from 1.25-5µg/mm² more preferably 3.0µg/mm². Paclitaxel is used for the lesions of peripheral arteries due to its lipophilic properties, short absorption and prolonged duration of antiproliferative effects. Paclitaxel is a natural cytotoxic anti proliferative agent with antimitotic properties originally used for cancer chemotherapy.

The excipient(s) may be selected from one or more of, without limitation salts and plasticizers. The salt(s) may be in the range of 30-35 weight %, while the plasticizer(s) may be in the range of 3-6 weight %. The salt helps in dispersion of the anti-proliferative drug from the surface of the balloon 1. The salt also prevents flaking of the coating upon dispersion while ensures minimal loss of drug in blood during the transit of the balloon from a puncture site to a treatment site. Further, the salt helps in releasing the drug in form of smaller particles hence, increasing the drug surface area for easy and speedy tissue uptake. Plasticizer helps prevent excessive loss of anti-proliferative drug during the transit of balloon from a puncture site to a treatment site. Plasticizer also prevents the coating surface from becoming dry.

Salts can be selected from urea, while plasticizers can be selected from one or more of, without limitation ethanol, triethyl citrate, tributyl citrate, acetyl tributyl citrate (ATBC), acetyl triethyl citrate, glycerol, dimethylsulfoxide, ethyl lactate, benzyl alcohol, benzylbenzoate, vitamin-E, tocopherol, dibutyl phthalate, dibutyl sebacate, glycerin, propylene glycol, polyethylene glycol (PEG).

According to the invention, urea is used as the salt. Urea is a colorless, odorless solid. Urea is highly soluble in water and practically non-toxic. Urea when dissolved in water is neither acidic nor alkaline. Due to its hydrophilic nature, urea easily and quickly dissolves in blood and the water content present in the tissues at the treatment site. Since, the anti-proliferative drug paclitaxel is highly hydrophobic, it is not easily released from the balloon surface. The use of urea with paclitaxel assists in the release of paclitaxel from the balloon surface. Once the drug is released from the balloon surface it resides on the tissue walls of the treatment site and is slowly embedded in the tissue, further stabilizing the spindle microtubules during cell mitosis, thereby arresting cell division and leading to cell death.

In an embodiment, PEG200 is be used as a plasticizer. PEG200 is a low molecular weight grade of polyethylene glycol. PEG200 is a clear, colorless, viscous liquid. PEG200 is soluble in water, acetone, glycols, benzene, glycerin and aromatic hydrocarbons. PEG due to its solubility in both polar and non-polar solvents acts as a dissolution aid for the anti-proliferative drug paclitaxel.

Optionally or additionally, a lubricant (or lubricity enhancer or humectant) may be added to the layer 23. The lubricant may be selected from one or more of, without limitation polyethylene glycols, glycerin, sorbitol etc. The lubricant improves uniformity of coating of the layer 23.

As represented in FIG.3 (a non-claimed embodiment), the outer surface 21 of the balloon 1 is coated with multiple layers. In an exemplary embodiment, the balloon 1 includes the following three layers: a base layer 25, a middle layer 27, and a top layer 29.

The base layer 25 acts as a support for the middle layer 27 and the top layer 29. The base layer 25 also ensures complete release of drug from the balloon 1. The base layer 25 ensures good adhesion of the middle layer 27. The base layer 25 may include without limitation a plasticizer and an adhesive binder. The plasticizer may be in the range of 8-12 weight %. The plasticizers may be selected from the plasticizers as described in FIG. 2. The plasticizers help to prevent excessive loss of anti-proliferative drug during the transit of balloon from a puncture site to a treatment site. Plasticizers also prevent the coating surface from becoming dry.

In an embodiment, ATBC may be used as the plasticizer. ATBC prevents excessive loss of anti-proliferative drug during the transit of the balloon from a puncture site to a treatment site. ATBC is a colorless liquid that is soluble in organic solvents.

The adhesive binder may be in the range of 0.5-0.8 weight %. The adhesive binders may be selected from one or more of, without limitation group of sugars, group of povidones, group of polysaccharides or polyvinyl pyrrolidone (PVP). The adhesive binders ensure adequate retention of the coating formulation over the balloon surface. The adhesion should be sufficient to prevent excessive loss of antiproliferative drug during the maneuvering of balloon catheter 100 to the target site.

The middle layer 27 includes, without limitation an anti-proliferative drug, an emulsifier, a stabilizer and a solvent. The anti-proliferative drug may be in the range of 40 to 60 weight %. The anti-proliferative drug may be selected from the antiproliferative drugs as described in FIG. 2.

The emulsifier may be in the range of 15-20 weight %. The emulsifiers may include, without limitation polysorbate 60, Fluronic F127, Poloxamer, phosphatidylcholine, glycerol monostearate, phospholipid or a combination thereof. The emulsifiers help in achieving higher drug encapsulation efficiency and higher cellular uptake which results in higher therapeutic effects.

The stabilizer may be in the range of 1-7 weight %. The stabilizers may be selected from one or more of, without limitation vitamin-E d-α-tocopheryl polyethylene glycol 1000 succinate (TPGS), sorbitan, group of polyxamers, and poly(vinyl)alcohol. The stabilizers help in increasing the drug encapsulation efficiency.

The components used in the middle layer 27 form a solid lipid nano-particle (SLN) suspension. In an embodiment, the SLN suspension using paclitaxel is prepared by using high speed homogenization and high pressure homogenization (elaborated in FIG. 4). The use of both the techniques ensures formation of nanoparticles preferably in the size range of 500-800 nanometers, more preferably less than 500nm.

The SLN suspension in the current embodiment may be selected from one or more excipients mentioned aforesaid, poloxamer as an emulsifier, vitamin-E TPGS as a stabilizer, water and dichloromethane as solvents.

In an embodiment of the present invention, the components of the top layer 29 and concentrations thereof are same as that of the base layer 25. Alternately, the components and concentration thereof of the top layer 29 may differ from the base layer 25.

The top layer 29 acts as a shield for the middle layer during for example, the wrapping process and storage. The top layer 29 also prevents drug loss during the transit of balloon 1 to the treatment site.

FIG. 4 depicts the process of preparation of SLN suspension. The process involves dissolving a mixture of Poloxamer and vitamin-E TPGS in water and/or dichloromethane. The solution formed is then subjected to high speed homogenization at 10000-16000 rpm for 10 minutes to 20 minutes, more preferably around 12000-15000rpm for duration of 15 minutes to 18 minutes. The process is conducted in a temperature controlled ice bath such that the temperature of the solution throughout the process is maintained below 20°C. The equipment may include without limitation, a temperature probe, propeller and its propeller shaft, etc.

Further, after 15-20 minutes, paclitaxel and dichloromethane are added sequentially at the rate of 1ml per 10 minutes under continuous high speed homogenization. The mixture is allowed to homogenize for another 60-120 minutes more preferably for 70-90 minutes. This homogenized solution is then subjected to high pressure homogenization.

High pressure homogenization is a reliable technique for the production of SLN suspension. In order to convert the particles into much smaller size, preferably less than 500 nm in size, the solution undergoes around 5-6 passes of high pressure homogenization.

The size of the particles of SLN suspension may be less than 500nm. The concentrations of the SLN suspension when mixed with the binder 90KD PVP may range from 0.20%-0.60%. The concentrations of the SLN suspension when mixed with the humectant PEG400 may range from 50%-70%.

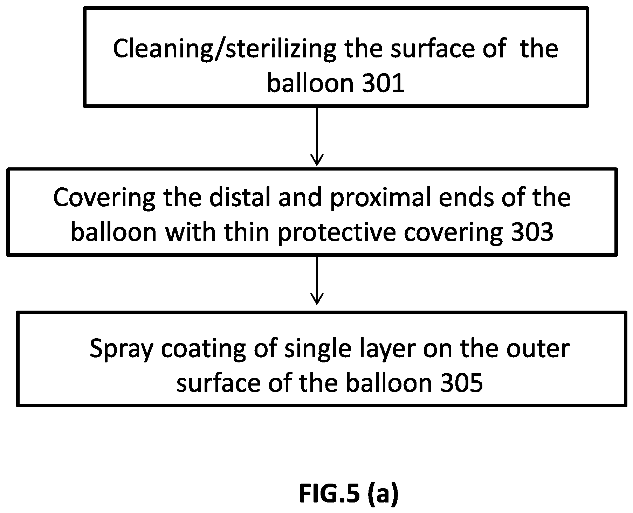

Fig.5 (a) illustrates a flow chart depicting the coating process on the outer surface 21 of the balloon 1 in the form of single layer. In an embodiment, the process is performed in a sterile environment. The coating process on the outer surface 21 of the balloon 1 commences at step 301. The outer surface 21 of the balloon 1 is cleaned/ sterilized by means of, without limitation iso-propyl alcohol or acetone.

At step 303, the distal end 17 and proximal end 19 of the balloon 1 are covered using a thin protective covering. The thin protective covering may be made of, without limitation, parafilm, nescofilm, molded polypropylene or teflon caps, etc. The thin protective covering may be in the form of molds with shapes corresponding to the distal and proximal ends 17, 19 of the balloon 1. Such molds may be placed over the proximal and distal ends 17, 19 of the balloon 1 prior to coating. The thin protective covering ensures uniform coating of the middle portion 15 of the outer surface 21 of the balloon 1. The thin protective covering may be easily detached from the balloon 1 post coating.

At step 305, the coating on the outer surface 21 of the balloon 1 takes place. As the distal end 17 and proximal end 19 of the balloon 1 are covered using a thin protective covering at step 303, coating is performed on the outer surface 21 corresponding to the middle portion 15 of balloon 1 only. The coating may be performed by means of, without limitation dip coating, pipette coating, syringe coating, spray coating, air or inert gas assisted spray coating, electrostatic spray coating or any other method known to those skilled in the art.

In an exemplary embodiment, the coating is done with the help of a spray coating equipment, for example, a spray gun. The parameters of operation of the coating equipment include distance between spray gun and balloon, rotation speed of balloon, nitrogen gas pressure inside the equipment, oscillation rate of the spray gun, temperature inside the equipment and flow rate of solution during coating process. The distance between spray gun and the balloon ranges from 3 cm to 6 cm and more preferably 4.5 cm to 5.5 cm. The rotation of balloon attached to collate ranges from 15 rpm to 20 rpm more preferably 17 rpm to 19 rpm. The pressure of nitrogen ranges from 0.5 to 1.5 kg/cm² more preferably 0.8 to 1.2 kg/cm². The oscillation rate of spray gun is between 30 rpm to 70 rpm more preferably between 50 to 60 rpm. The flow rate of solution during coating process is 0.1 to 0.5 ml/min more preferably 0.15 to 0.25 ml/min. The temperature at which the coating process takes place is 22±3°C.

The balloon 1 is coated under controlled humidity. In an embodiment, the humidity of the coating environment is 45% relative humidity (RH) to achieve a uniform coating on the outer surface 21 of the balloon 1. The said value of humidity ensures that the outer surface 21 of the balloon 1 is neither too dry nor too sticky. Therefore, no cracks are formed on the outer surface 21 of the balloon 1 after pleat-fold.

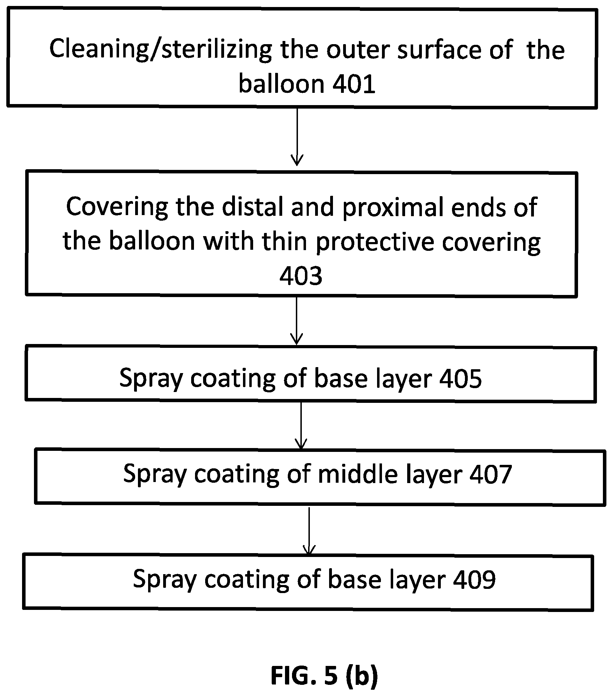

Fig.5 (b) (a non-claimed embodiment) illustrates a flow chart depicting the process of coating of three layers on the outer surface 21 of the balloon 1. In an embodiment, the process is performed in a sterile environment. In an embodiment, the steps of cleaning and covering ends of the balloon 401 and 403 respectively in the context of coating process for three layers are same as the steps 301 and 303 of the coating process for single layer as illustrated in Fig.5 (a).

At step 405, the base layer 25 is coated over the middle portion 15 of the outer surface 21 of the balloon 1. The base layer 25 is dried for a period of 5-20 minutes.

At step 407, the middle layer 27 is coated over the base layer 25. The middle layer 27 is dried for a period of 5-20 minutes.

At step 409, the top layer 29 is coated over the middle layer 27 forming a three layer coating. The base layer 40 is dried for a period of 5-20 minutes.

In an exemplary embodiment, the coatings at steps 405, 407 and/or 409 are performed with the help of a spray coating equipment, for example, a spray gun. In an embodiment, the parameters of operation of the coating equipment for coating the base layer 25, the middle layer 27 and the top layer 29 are same as the parameters of operation of the coating equipment for coating single layer. This technique of coating ensures a uniform distribution of drug on the outer surface 21 of the balloon 1. This technique results in a uniform release of drug during the dispersion of the balloon 1 by inflation.

Optionally/additionally, the drug coated balloon of the present invention is wrapped or pleated and/or folded after the coating process. The drug coated balloon is then sterilized. The drug coated balloon after pleat-fold may then be employed for various testing. In an embodiment, the drug coated balloon after pleat-fold is tested for drug elution profile and pharmacokinetics at different time intervals up to approximately 30 days in an animal model. The concentrations of drug were measured in blood, artery tissues and residual drug on balloon. The blood concentrations were highest within 3-5 hours of implantation and progressively declined up to 48 hours. The peak concentrations of drug in arterial wall were seen at 24 hours but persisted up to 28 days.

The present invention is explained with the help of following examples. Reference Example 1: The layer 23 was coated on the outer surface 21 of the balloon 1 in the form of a single layer. Table 1 shows the composition of formulation A1. As shown below, there were no flakes observed on balloon coated with A1 on dispersion. The drug content was also checked by high performance liquid chromatography (HPLC). The drug content of A1 was found to be between 60-80%.

**Table 1**

| **Sample** | **Paclitaxel (wt %)** | **ATBC (wt %)** | **Urea (wt %)** | **PEG200 (wt %)** | **Glycerol (wt %)** | **Flakiness** |
|---|---|---|---|---|---|---|
| A1 | 61.81 | 0.25 | 11.32 | 22.08 | 04.52 | No flakes |

Example 2: The layer 23 having a composition B1 was coated on the outer surface 21 of the balloon 1 in the form of a single layer. B1 comprises of paclitaxel having a concentration of 63.19%, urea having a concentration of 31.945 and PEG200 having a concentration of 4.86%. The formulation B1 is observed to have no surface damage after pleating-folding even with reduced balloon crossing profile and sterilization. A uniform dispersion with minute particles was observed on dispersing the coated balloon 1 in dispersing media. The drug content by HPLC was between 85-95%.

Reference Example 3: The outer surface 21 of the balloon 1 was coated with the base layer 25, the middle layer 27 and the top layer 29. The middle layer 27 consisted of polyethylene glycol as humectant. The constituents of the base layer 25 and the top layer 29 were same. The base layer 25 and top layer 29 consisted of 90KD PVP as binder and dichloromethane as solvent. The concentration of 90KD PVP in dichloromethane present in the base layer 25 and the top layer 30 was 0.7%. The concentration of humectant in the middle layer 27 was varied to produce the following formulation ( F1) :
F1: 3.6 mg/ml 90KD PVP in nano + PEG-400 stock solution-0.36% + 60%

The balloons coated with F1 formulation had relatively good balloon surface after pleat-fold process.

Reference Example 4: The outer surface 21 of the balloon 1 was coated with three layers namely the base layer 25, the middle layer 27 and the top layer 29. The following formulations shown in Table 2 shows addition of plasticizer ATBC in different layers.

**Table 2**

| **Formulation** | | **Constituents** | **Concentration** |
|---|---|---|---|
| **G1** | **Base Layer** | 90KD PVP | 0.7% |
| | **Middle Layer** | 90KD PVP in nano + PEG-400 stock solution | 0.36% + 60% |
| | **Top Layer** | 90KD PVP + ATBC in DCM | 0.7% + 10% |
| | | | |
| **G2** | **Base Layer** | 90KD PVP + ATBC in DCM | 0.7% +10% |
| | **Middle Layer** | 90KD PVP in nano + PEG-400 stock solution | 0.36% + 60% |
| | **Top Layer** | 90KD PVP | 0.7% |
| | | | |
| **G3** | **Base Layer** | 90KD PVP | 0.7% |
| | **Middle Layer** | 90KD PVP in nano + PEG-400 stock solution+ ATBC in DCM | 0.36 % + 60% + 10% |
| | **Top Layer** | 90KD PVP | 0.7% |
| | | | |
| **G4** | **Base Layer** | 90KD PVP + ATBC in DCM | 0.7% + 10% |
| | **Middle Layer** | 90KD PVP in nano + PEG-400 stock solution | 0.36% + 60% |
| | **Top Layer** | 90KD PVP + ATBC in DCM | 0.7% + 10% |

The balloons coated with formulation G1 and G2 had minor damages to the coating surface after pleating and folding. However, the balloons coated with formulation G3 illustrated peeling of coating layer from the balloon surface during the pleating-folding process. The balloons coated as per formulation G4 showed comparatively better surface finish after pleating-folding with good dispersion in the dispersion media with the size of nano-particles. Therefore, addition of ATBC in base layer 25 as well as top layer 29 showed better results with uniform dispersion and reduced profile.

The scope of the invention is only limited by the appended patent claims. More generally, those skilled in the art will readily appreciate that all parameters, dimensions, materials, and configurations described herein are meant to be exemplary and that the actual parameters, dimensions, materials, and/or configurations will depend upon the specific application or applications for which the teachings of the present invention is/are used.

## Claims

1. A drug coated balloon comprising:
a balloon having an outer surface, the outer surface coated with a single layer of a solution of an anti-proliferative drug, the single layer coating comprising:
at least one anti-proliferative drug present in the concentration of 60-70 weight %; and,
at least two excipients, the at least two excipients include urea present in the concentration of 30-35 weight % and at least one plasticizer present in the concentration of 3-6 weight %;
wherein the urea disperses the anti-proliferative drug from a surface of the balloon and prevents flaking of the single layer coating of the anti-proliferative drug upon dispersion, wherein the at least one plasticizer prevents excessive loss of at least one anti-proliferative drug during transit of the balloon from a puncture site to a treatment site, the at least one plasticizer prevents the single layer of the antiproliferative drug from drying.

2. The drug coated balloon as claimed in claim 1 wherein the anti-proliferative drug is selected from one or more of sirolimus, biolimus, zotarolimus, everolimus, paclitaxel, vinblastine, vindesine or vincristine.

3. The drug coated balloon as claimed in claim 1 wherein the single layer of the antiproliferative drug includes a lubricant.

4. The drug coated balloon as claimed in claim 1 wherein the lubricant is selected from polyethylene glycols, glycerin, or sorbitol.

5. The drug coated balloon as claimed in claim 1 wherein the plasticizer is selected from ethanol, triethyl citrate, tributyl citrate, acetyl tributyl citrate, acetyl triethyl citrate, glycerol, dimethylsulfoxide, ethyl lactate, benzyl alcohol, benzylbenzoate, Vitamin E, tocopherol, dibutyl phthalate, dibutyl sebacate, glycerin, propylene glycol or polyethylene glycol.

## Patentansprüche

1. Medikamentenbeschichteter Ballon, umfassend:
einen Ballon, der eine Außenfläche aufweist, wobei die Außenfläche mit einer einlagigen Beschichtung einer Lösung eines antiproliferativen Medikaments beschichtet ist, wobei die einlagige Beschichtung umfasst:
mindestens ein antiproliferatives Medikament, das in der Konzentration von 60-70 Gewichtsprozent vorliegt; und
mindestens zwei Hilfsstoffe, wobei die mindestens zwei Hilfsstoffe Harnstoff in der Konzentration von 30-35 Gewichtsprozent und mindestens einen Weichmacher in der Konzentration von 3-6 Gewichtsprozent beinhalten;
wobei der Harnstoff das antiproliferative Medikament von einer Oberfläche des Ballons dispergiert und Abblättern der einlagigen Beschichtung des antiproliferativen Medikaments beim Dispergieren verhindert, wobei der mindestens eine Weichmacher einen übermäßigen Verlust mindestens eines antiproliferativen Medikaments während eines Transports des Ballons von einer Punktionsstelle zu einer Behandlungsstelle verhindert und der mindestens eine Weichmacher Austrocknen der einlagigen Beschichtung des antiproliferativen Medikaments verhindert.

2. Medikamentenbeschichteter Ballon nach Anspruch 1, wobei das antiproliferative Medikament aus einem oder mehreren von Sirolimus, Biolimus, Zotarolimus, Everolimus, Paclitaxel, Vinblastin, Vindesin oder Vincristin ausgewählt ist.

3. Medikamentenbeschichteter Ballon nach Anspruch 1, wobei die einlagige Beschichtung des antiproliferativen Medikaments ein Gleitmittel beinhaltet.

4. Medikamentenbeschichteter Ballon nach Anspruch 1, wobei das Gleitmittel aus Polyethylenglykolen, Glycerin oder Sorbit ausgewählt ist.

5. Medikamentenbeschichteter Ballon nach Anspruch 1, wobei der Weichmacher aus Ethanol, Triethylcitrat, Tributylcitrat, Acetyltributylcitrat, Acetyltriethylcitrat, Glycerol, Dimethylsulfoxid, Ethyllactat, Benzylalkohol, Benzylbenzoat, Vitamin E, Tocopherol, Dibutylphthalat, Dibutylsebacat, Glycerin, Propylenglykol oder Polyethylenglykol ausgewählt ist.

## Revendications

1. Ballonnet enrobé de médicament comprenant :
un ballonnet présentant une surface externe, la surface externe étant recouverte d'une seule ouche d'une solution d'un médicament antiprolifératif, la seule couche comprenant :
au moins un médicament antiprolifératif présent à une concentration de 60-70 % en poids ; et,
au moins deux excipients, les au moins deux excipients incluent de l'urée présente à une concentration de 30-35 % en poids et au moins un plastifiant présent à une concentration de 3-6 % en poids ;
dans lequel l'urée disperse le médicament antiprolifératif à partir d'une surface du ballonnet et empêche l'écaillage du revêtement de seule couche du médicament antiprolifératif lors de la dispersion, dans lequel le au moins un plastifiant empêche une perte excessive d'au moins un médicament antiprolifératif pendant le transit du ballonnet depuis un site de ponction jusqu'à un site de traitement, et dans lequel le au moins un plastifiant empêche la seule couche du médicament antiprolifératif de se dessécher.

2. Ballonnet enrobé de médicament selon la revendication 1, dans lequel le médicament antiprolifératif est choisi parmi un ou plusieurs du sirolimus, du biolimus, du zotarolimus, de l'évérolimus, du paclitaxel, de la vinblastine, de la vindésine ou de la vincristine.

3. Ballonnet enrobé de médicament selon la revendication 1, dans lequel la seule couche du médicament antiprolifératif inclut un lubrifiant.

4. Ballonnet enrobé de médicament selon la revendication 1, dans lequel le lubrifiant est choisi parmi les polyéthylène glycols, la glycérine ou le sorbitol.

5. Ballonnet enrobé de médicament selon la revendication 1, dans lequel le plastifiant est choisi parmi l'éthanol, le citrate de triéthyle, le citrate de tributyle, le citrate d'acétyltriéthyle, le glycérol, le diméthylsulfoxyde, le lactate d'éthyle, l'alcool benzylique, le benzoate de benzyle, la vitamine E, le tocophérol, le phtalate de dibutyle, le sébacate de dibutyle, la glycérine, le propylène glycol ou le polyéthylène glycol.
